(19) Europäisches Patentamt / European Patent Office / Office européen des brevets

(11) **EP 3 691 688 B1**

(12) **EUROPEAN PATENT SPECIFICATION**

(45) Date of publication and mention
of the grant of the patent:
**08.12.2021 Bulletin 2021/49**

(21) Application number: **18782983.3**

(22) Date of filing: **05.10.2018**

(51) Int Cl.:
*A61K 47/32* (2006.01)    *A61K 9/00* (2006.01)
*A61K 47/02* (2006.01)    *A61K 33/00* (2006.01)
*A61Q 19/00* (2006.01)

(86) International application number:
**PCT/EP2018/077120**

(87) International publication number:
**WO 2019/068858 (11.04.2019 Gazette 2019/15)**

(54) **HYDROGEL COMPOSITIONS FOR THE TREATMENT OF MOLLUSCUM CONTAGIOSUM**

HYDROGELZUSAMMENSETZUNG ZUR BEHANDLUNG VON MOLLUSCUM CONTAGIOSUM

COMPOSITIONS D'HYDROGEL POUR LE TRAITEMENT DU MOLLUSCUM CONTAGIOSUM

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.10.2017 EP 17382671**

(43) Date of publication of application:
**12.08.2020 Bulletin 2020/33**

(73) Proprietor: **Laboratorio Reig Jofré, S.A.
08970 Sant Joan Despí (ES)**

(72) Inventors:
• **GONZÁLEZ PLÁGARO, Jordi
08970 Sant Joan Despí (ES)**

• **NAVARRO PUJOL, Francisco
08970 Sant Joan Despí (ES)**
• **FERNÁNDEZ CAMPOS, Francisco
08970 Sant Joan Despí (ES)**
• **NIETO ABAD, Carlos
08970 Sant Joan Despí (ES)**

(74) Representative: **ZBM Patents - Zea, Barlocci & Markvardsen
Rambla Catalunya, 123
08008 Barcelona (ES)**

(56) References cited:
**EP-A1- 2 471 541      WO-A1-2016/007692
CN-B- 101 433 617**

EP 3 691 688 B1

## Description

## Technical Field

The present invention relates to a topical composition in the form of a hydrogel containing a metal hydroxide, and to a process for its preparation. It also relates to the use of this composition in the treatment of *Molluscum Contagiosum* and related diseases.

## Background Art

*Molluscum Contagiosum* (MC) is a viral infection of the skin caused by a DNA poxvirus called the *Molluscum Contagiosum* Virus (MCV). MC occurs most commonly in children aged from one to ten. About one out of six children are infected at some time.

MC can affect any area of the skin but is most common on the trunk of the body, arms, and legs. It is characterised by the appearance of lobulated epidermal lesions on the skin as a result of excessive cellular proliferation stimulated in the keratinocyte layer by the virus. These lesions are generally not painful, but they may itch or become irritated and scratching them may lead to further infection or scarring. Since there is no permanent immunity to the virus, it is possible to become infected more than once.

Although the lesions caused by MC may clear up on its own in some cases, untreated lesions can reach large sizes and spontaneous remission can last up to 6 months or longer. Therefore, treatment is generally recommended also in order to alleviate discomfort (itching and burning), improve clinical appearance, and reduce autoinoculation or transmission to other people, since as long as the skin growths are present, there is a possibility of transmitting the infection.

There are many existing treatments for *Molluscum Contagiosum.* One of these consists in removing the lesions by surgical intervention (including e.g. cryosurgery or laser). However, these treatments are generally rather ineffective and unsuitable for children because they can be painful and/or may lead to residual scarring. Besides, these treatments cannot be applied at home, and often require multiple visits to the doctor making it difficult and impractical to follow the therapy. On the other hand, systemic treatments can be expensive and give rise to undesirable side-effects.

Furthermore, the application of topical agents for the treatment of MC has been also used for years. These treatments consist in the application for example of organic acids, such as glacial acetic acid and/or salicylic acid and lactic acid, or bases such as potassium hydroxide, calcium hydroxide and sodium hydroxide and have the advantage that can be applied at home. However, these agents are skin-irritating and when administered in the form of solutions it is difficult to limit their application locally to the affected area causing discomfort in the neighboring healthy skin.

For example, topical compositions based on sodium hydroxide and/or potassium hydroxide for the treatment of *Molluscum Contagiosum* have been described in EP2471541. This document discloses medicines which comprise from 1.0-60.0% of sodium hydroxide and/or potassium hydroxide, from 0.25-1.0% of an anesthetic agent, and 0.01-0.5% of carbomer 941 as excipient.

Similarly, CN101433617 relates to a cream for dissolving *Molluscum Contagiosum* which is also based on sodium hydroxide or potassium hydroxide (2-60%), and further comprises 0-3.0% of licorice, 0-3.0% 0-3.0 skullcap, 0-3.0% Cork, 0-3.0% rhubarb, 0-1.0% menthol, 0-10% calcium hydroxide, and 0.75-1.0% hydrochloric acid levobupivacaine (or Toad, Bupivacaine hydrochloride, lidocaine, tetracaine or procaine). The cream may additionally comprise 0-1.0% of carbomer 941.

However, in the hand of the inventors, compositions of EP2471541 and CN101433617 showed to be unstable over time due to the appearance of KOH crystals in the formulation. Besides these compositions did not have gel consistency, therefore making it difficult their localized application on the lesion only.

From what is known in the art it is derived that there is still the need of providing pharmaceutical compositions for the treatment of *Molluscum Contagiosum* that overcome the problems of the prior art treatments, in particular in terms of efficacy, stability, and patient compliance.

## Summary of Invention

The inventors have developed a topical composition in the form of a hydrogel for the treatment of viral infections such as *Molluscum Contagiosum* that can incorporate a high amount of a metal hydroxide (6 to 15% by weight of the total composition) while maintaining its physical and chemical stability. As illustrated by the examples of the invention, and unlike in other tested prior art compositions, the metal hydroxide does not crystallize from the compositions of the invention over time. In fact, the content of the metal hydroxide that is responsible for the therapeutic effect does not significantly change during storage. Thus, the therapeutic profile and efficacy of the product can be maintained for a long period and the minimum durability date of the composition is extended.

Moreover, the composition of the invention has gel consistency which is advantageous because when the formulation is applied it remains on the application site to be treated increasing its residence time on the lesion. Furthermore, the gel consistency avoids that the composition reaches neighboring healthy skin which could cause irritation and discomfort. The viscosity of the composition at room temperature further allows its administration thorough a device such as a pen-type applicator, which facilitates administration and patient compliance, also in children, reduces the likelihood of discontinued therapy, and improves the cure rate. The gels of the invention have the additional advantages of adhering to irregular lesion contours such as the ones caused by e.g. *Molluscum Contagiosum,* and easy application and removal. Furthermore, if desired, these gels may contain further components that provide a cooling feeling when applied to a lesion so that the patient discomfort can be reduced and the acceptance of the formulation be increased.

Finally, the composition of the invention can be prepared by a simple process which is suitable for industrialization.

Thus, a first aspect of the present invention refers to a topical pharmaceutical or veterinary composition in the form of a hydrogel comprising:

a) a crosslinked polymer of acrylic acid in an amount from 1 to 4% by weight with respect to the total weight of composition; and
b) a metal hydroxide selected from sodium hydroxide and potassium hydroxide in an amount from 6 to 15% by weight with respect to the total weight of composition.

Another aspect of the invention relates to a device comprising the topical pharmaceutical or veterinary composition as defined above.

Another aspect of the invention relates to the topical pharmaceutical or veterinary composition as previously defined, for use in the treatment of a viral-induced lesion resulting from a viral infection.

**Brief Description of Drawings**

Fig. 1 shows the viscosity (mPa·s) vs shear rate (s$^{-1}$) of the hydrogel of example 8.
Fig. 2 shows the viscosity (mPa·s) vs shear rate (s$^{-1}$) of the formulation of comparative example 1.
Fig. 3 shows the viscosity (mPa·s) vs shear rate (s$^{-1}$) of the formulation of comparative example 2.

**Detailed description of the invention**

All terms as used herein in this application, unless otherwise stated, shall be understood in their ordinary meaning as known in the art. Other more specific definitions for certain terms as used in the present application are as set forth below and are intended to apply throughout the description and claims.

Unless otherwise stated, all percentages mentioned herein regarding the components of the composition are expressed in weight with respect to the total weight of the composition, provided that the sum of the amounts of the components is equal to 100%.

For the purposes of the invention, the term "about" refers to a range of values ± 10% of a specified value. For example, the expression "about 20" includes ± 10% of 20, i.e. from 18 to 22.

As used herein, "viscosity" refers to a measure of the resistance of a fluid to deform under shear stress and describe the fluid's internal resistance to flow and can be measured as a function of the shear rate by using a rheometer. It is generally expressed in Pascalseconds (Pa·s). For example, the rheological test may be carried out in a Brookfield dv-III ultra apparatus, using spindle SC4-28 at 100 rpm, 20 °C, atmospheric pressure, for 10 min (method 1); or alternatively using spindle SC4-21 at 100 rpm, 20 °C, atmospheric pressure, for 10 min (method 2); or alternatively spindle RV7 at 100 rpm, 20 °C, atmospheric pressure, for 10 min (method 3).

The term "pH" is defined as the value given by a suitable, properly standardized, potentiometric sensor and measuring system. The pH of the compositions disclosed herein is measured by well-known methods in the art. For example, the pH can be measured with using a Metrohm pHmeter. Normally, the sample is firstly diluted in water (1/10, i.e. 1 g is disolved up to 10 mL of water).

For the purposes of the invention, room temperature is 20-25 °C.

As mentioned above, the invention relates to a topical composition in the form of a hydrogel comprising a crosslinked polymer of acrylic acid and a metal hydroxide in specific amounts.

For the purposes of the present invention, the term "topical" refers to the local administration of the composition (other than systemic, i.e., parenteral and enteral) to the skin or scalp.

The term "gel" as used herein means a semi-solid system having a solid phase dispersed in a liquid phase, wherein the solid phase is the discontinuous phase and the liquid is the continuous phase. For the purposes of the invention having a gel consistency means that it can be administered through a device such as a in pen-type applicators without dripping. The term "hydrogel" as used herein refers to a three-dimensional network of hydrophilic polymer chains that are capable of absorbing and retaining large quantities of water (more than 99.9% water) to form a stable structure. Generally, the hydrogel of the invention has a viscosity from 700 to 20000 mPa·s, more particularly from 700-10000 mPa·s, and even more particularly from 700 to 1500 mPa·s as measured by method 1 (SC4-28) as described above and/or has a viscosity from 200 to 10000 mPa·s, more particularly from 200-5000 mPa·s, and even more particularly from 200 to 800 mPa·s as measured by method 2 (SC4-21) as described above . Hydrogels having a viscosity from 700 to 20000 mPa·s as measured by method 1 (SC4-28) and/or from 200 to 10000 mPa·s as measured by method 2 (SC4-21) are especially suitable to be used in pen-type applicators.

Semi-solid forms such as the hydrogels of the invention can be further characterized by parameters such as hardness, adhesiveness, cohesiveness, and elasticity for example determined by Texturometric analysis.

The term "hardness" as used herein refers to the force required to obtain a deformation of a composition. It is generally expressed in Newtons (N). This parameter is calculated from the peak force of the first compression of the composition in either the Texture Profile Analysis (TPA) assay or the compression assay. The term "adhesiveness" as used herein refers to a TPA parameter that quantifies a material's tendency to adhere to the probe. It is generally expressed in Joules (J). This parameter is calculated by determining the area under the force-displacement curve during the extension. The term "cohesiveness" as used herein refers to a TPA parameter that is calculated from the area of work during the first compression of the composition. It is thought to express the structural integrity of composition, and refers to a property characterized by the strength of internal bonding that makes up the body of a composition. It can be determined as the ratio between the positive area obtained during the understanding stage in the first and second cycles and represents the speed with which a material is reorganized after being subjected to a mechanical action. The term "elasticity" as used herein is intended to mean the rate at which deformed compositions go back to their original undeformed state after removal of the force. It is generally expressed in Newton/meter (N/m). This parameter is calculated from the slope of the straight-line displacement force during the penetration stage of the probe in the sample. It can be measured in different cycles.

In one embodiment, optionally in combination with one or more features of the various embodiments described above or below, the composition of the invention has a hardness value from 0.04 to 0.10 N; more particularly about 0.07 N.

In another embodiment, optionally in combination with one or more features of the various embodiments described above or below, the composition of the invention has an adhesiveness value from 55 to 75 J; more particularly about 56 J.

In another, optionally in combination with one or more features of the various embodiments described above or below, the composition of the invention has a cohesiveness from 0.5 to 1.1; more particularly about 0.98.

In another, optionally in combination with one or more features of the various embodiments described above or below, the composition of the invention has an elasticity from 6 to 13 N/m; more particularly about 9.5 N/m.

As mentioned above, the composition of the invention comprises a crosslinked polymer of acrylic acid that is a carbomer. Generally, the crosslinked polymer of acrylic acid is a hydrophilic polymer that is dispersible in water and is capable of forming a gel.

For the purposes of the invention the terms "crosslinked polymer of acrylic acid" "carbomer", "carboxyvinyl polymer" and "carbopol" are used interchangeably. The term "carbomer" as used herein refers to a homo- or copolymer of acrylic acid that is crosslinked with polyalkenyl ethers or divinyl glycol, e.g. with an allyl ether of pentaerythritol (allyl pentaerythritol) or an allyl ether of sucrose (allyl sucrose), or an allyl ether of propylene. Copolymers are formed e.g. with minor levels of long chain alkyl acrylate comonomers. Carbomers are sold for example by the B.F. Goodrich Company and The Lubrizol Corporation under the trade mark of Carbopol. There are a variety of carbomer polymer grades which differ in their performance and characteristics. These grades are distinguished by a number designation following the product name.

Carbopols can also be classified as carbopol homopolymers, carbopol copolymers, and carbopol interpolymers. Carbopol homopolyers are polymers of acrylic acid crosslinked with allyl sucrose or allyl pentaerythritol. Carbopol copolymers are polymers of acrylic acid and $C_{10}$-$C_{30}$ alkyl acrylate crosslinked with allyl pentaerythritol. Carbopol interpolymers are a carbomer homopolymer or copolymer that contains a block copolymer of polyethylene glycol and a long chain alkyl acid ester.

Non-limiting examples of crosslinked polymer of acrylic acid that can be used in the present invention include carbopol 941 NF, carbopol 971 NF, carbopol 974 NF and carbopol 980 NF.

In one embodiment, optionally in combination with one or more features of the various embodiments described above or below, the crosslinked polymer of acrylic acid is a homopolymer. More particularly, the polymer of acrylic acid is crosslinked with allyl sucrose or allyl pentaerythritol, even more particularly, with allyl pentaerythritol. Preferably, the carbomers are water-soluble.

In another embodiment, optionally in combination with one or more features of the various embodiments described above or below, the crosslinked polymer of acrylic acid has a viscosity from 40 to 60 Pa·s measured in Brookfield RTV viscometer at 20 rpm, 25 °C, atmospheric pressure, in a 0.5 % weight/weight water dispersion mucilage neutralized to pH 7.3-7.8 (spindle number 7). More particularly, the crosslinked polymer of acrylic acid is carbomer 980, marketed under the trade name Carbopol 980 NF.

In another embodiment, optionally in combination with one or more features of the various embodiments described above or below, the crosslinked polymer of acrylic acid has a viscosity from 4 to 10 Pa·s; measured in Brookfield RTV viscometer at 20 rpm, 25 °C, atmospheric pressure, in a 0.5 % weight/weight water dispersion mucilage, neutralized to pH 7.3-7.8 (spindle number 5). More particularly the crosslinked polymer of acrylic acid is carbomer 941, marketed under the trade name Carbopol 941 NF.

In another embodiment, optionally in combination with one or more features of the various embodiments described above or below, the crosslinked polymer of acrylic acid has a viscosity from 4 to 11 Pa·s; measured in Brookfield RTV viscometer at 20 rpm, 25 °C, atmospheric pressure, in a 0.5 % weight/weight water dispersion mucilage, neutralized to pH 7.3-7.8 (spindle number 5). More particularly the crosslinked polymer of acrylic acid is carbomer 971, marketed under the trade name Carbopol 971 NF.

In another embodiment, optionally in combination with one or more features of the various embodiments described above or below, the crosslinked polymer of acrylic acid has a viscosity from 29.4 to 39.4 Pa·s; measured in Brookfield RTV viscometer at 20 rpm, 25 °C, atmospheric pressure, in a 0.5 % weight/weight water dispersion mucilage, neutralized to pH 7.3-7.8 (spindle number 6). More particularly the crosslinked polymer of acrylic acid is carbomer 974, marketed under the trade name Carbopol 974 NF.

The crosslinked polymer of acrylic acid as defined above is present in the composition of the invention in an amount from 1 to 4%, more particularly from 1.5 to 4%, by weight with respect to the total weight of composition. In one embodiment, optionally in combination with one or more features of the various embodiments described above or below, the crosslinked polymer of acrylic acid is present in an amount about 1%, about 1.5%, about 2%, about 3%, or about 4% by weight, more particularly about 2% by weight, with respect to the total weight of composition.

The composition of the invention also comprises a metal hydroxide. In one embodiment, optionally in combination with one or more features of the various embodiments described above or below, the metal hydroxide is selected from sodium hydroxide, potassium hydroxide and calcium hydroxide. More particularly, the metal hydroxide is potassium hydroxide.

The metal hydroxide is present in the composition of the invention in an amount from 6 to 15%, more particularly from 8 to 15%, by weight with respect to the total weight of composition. This amount is to be understood herein as the amount of metal hydroxide present in the final composition and it can be determined by titration by methods well-known in the art. For example when about 1 g of sample is used for the measurement; this amount is diluted in water (20 mL); and 15 mL of the diluted solution are titrated with HCl 0.1 N in a Crison Compact titrator (Cod.: 950008, electrode S2-02), the content of KOH (%) can be determined by the following formula:

$$\text{Content KOH (\%)} = \text{Conc*volume*7.4817}$$

wherein "conc" is the exact formulation concentration (in g/mL) of the sample (in this case 1 g/20 mL) and "volume" is the amount of HCl 0.1 N (in mL) added until the titration curve shows an inflection point.

In one embodiment, optionally in combination with one or more features of the various embodiments described above or below, the metal hydroxide is present in an amount from 9 to 12%, or about 6%, about 7%, about 8%, or about 9%, or about 10%, or about 11%, or about 12%, or about 13%, or about 14%, or about 15% by weight, more particularly about 10% by weight, with respect to the total weight of composition.

In the composition of the invention the weight ratio between the metal hydroxide and the crosslinked polymer of acrylic acid is from 1:2 to 1:12. In one embodiment, optionally in combination with one or more features of the various embodiments described above or below, the weight ratio between the polymer and the metal hydroxide is from 1:2 to 1:10, more particularly from 1:3 to 1:7, and even more particularly is about 1:5.

The compositions of the invention are stable (i.e. do not crystallize) over a period of time equal to or higher than 15 months, more particularly equal or higher than 2 years, 3 years or 5 years.

The topical composition of the invention may comprise further pharmaceutically or veterinary acceptable excipients and/or carriers. Thus, in one embodiment, optionally in combination with one or more features of the various embodiments described above or below, the topical composition previously defined further comprises additional pharmaceutically or veterinary acceptable excipients and/or carriers.

The appropriate excipients and/or carriers, and their amounts, can readily be determined by those skilled in the art according to the type of formulation being prepared.

The expression "pharmaceutically or veterinary acceptable excipients and/or carriers" means that the excipients or carriers are suitable for the preparation of compositions for pharmaceutical or medical uses in humans and animals.

Each component must be pharmaceutically or veterinary acceptable in the sense of being compatible with the other ingredients of the pharmaceutical or veterinary composition. It must also be suitable for use in contact with tissues or organs of humans and animals without excessive toxicity, irritation, allergic response, immunogenicity or other problems or complications consistent with a reasonable risk/benefit relationship.

Non-limiting examples of excipients and/or carriers that can be used in the compositions of the invention include solvents, cooling/refreshing agents, skin protectants, local anesthetics, surfactants, emulsifiers, emollients, preservatives and combinations thereof.

Non-limiting examples of solvents that can be used include the $(C_2-C_3)$alcohols, water, acetone, hexylene glycol, terpenes, di-terpenes, tri- terpenes, terpenols, limonene, terpene-ol, 1 -menthol, dioxolane, sulfoxides, such as dimethylsulfoxide (DMSO), methyl dodecyl sulfoxide, dimethylacetamide, monooleate of ethoxylated glycerides (with 8 to 10 ethylene oxide units), azone (1-dodecylazacyclo-heptan-2-one), 2-(n-nonyl)-1,3-dioxolane, esters, such as isopropyl myristate/palmitate, ethyl acetate, butyl acetate, methyl proprionate, capric/caprylic triglycerides, octylmyristate, dodecyl-myristate; myristyl alcohol, lauryl alcohol, lauric acid, lauryl lactate ketones; amides, such as acetamide oleates such as triolein; various alkanoic acids such as caprylic acid; alkanols, such as dialkylamino acetates, and admixtures thereof. For the purposes of the invention, the term "$(C_2-C_3)$alcohol" refers to a hydrocarbon chain in which one or more hydrogen atoms have been replaced by one or more hydroxy groups. Non-limiting examples of "$(C_2-C_3)$alcohols include: aliphatic alcohols such as ethanol, 1-propanol, 2-propanol (i.e. isopropanol), dihydroxy alcohols such as ethylene glycol, propylene glycol; or trihydroxy alcohols such as glycerin.

In one embodiment, optionally in combination with one or more features of the various embodiments described above or below, the amount of water in the composition is from 40-95%, more particularly from 60 to 95% by weight with respect to the total weight of composition.

In another embodiment, optionally in combination with one or more features of the various embodiments described above or below, the composition comprises an additional solvent, more particularly a water miscible solvent. More particularly, the additional solvent is a $(C_2-C_3)$alcohol. The use of an alcohol may facilitate the dissolution of further components of the composition such as e.g. anti-irritating agents and thus, make the process for the preparation of the composition much simpler and easier for industrialization. The solvents can also be useful for enhancing the penetration of the active agent.

In another embodiment, optionally in combination with one or more features of the various embodiments described above or below, the $(C_2-C_3)$alcohol is selected from the group consisting of ethanol, 1-propanol, isopropanol, ethylene glycol, propylene glycol, glycerin and combinations thereof. More particularly, the $(C_2-C_3)$alcohol is ethanol or isopropanol. More particularly, the $(C_2-C_3)$alcohol is present in an amount from 0.5 to 15 %, even more particularly from 1 to 10, and even more particularly about 3% by weight, with respect to the total weight of composition.

In another embodiment, optionally in combination with one or more features of the various embodiments described above or below, the weight ratio between the alcohol and the crosslinked polymer of acrylic acid is from 10:1 to 1:1, more particularly from 5:1 to 1:1, more particularly is about 3:2.

In another embodiment, optionally in combination with one or more features of the various embodiments described above or below, the weight ratio between the metal hydroxide and the alcohol is from 15:10 to 8:1, more particularly from 2:1 to 5:1, more particularly is about 10:3.

Cooling/refreshing agents may provide on or following application to a body surface a decrease in the burning or itching sensation that the metal hydroxide could cause. Non-limiting examples of cooling/refreshing agents include menthol or an isomer thereof, a menthol derivative such as menthol ethylene glycol carbonate (Frescolat® type MGC), menthol Propylene Glycol Carbonate (Frescolat® type MPC), menthyl lactate (Frescolat ML®) and Menthone Glycerin Acetal (Frescolat MGA®); camphor, aloe vera, comfrey, cardamone, eucalyptus, peppermint, hyssop, rosemary, and the like.

Skin protectants may soothe the skin as well as maintain the integrity of the skin to prevent additional damage. Non-limiting examples of skin protectants include symsitive (INCI name Pentylene Glycol, 4-t-Butylcyclohexanol marketed by Symrise); allantoin, cocoa butter, dimethicone, kaolin, shark liver oil, petrolatum, lanolin, vegetable oils, ethoxylated oils and lipids, polyalkylene oxides, polyvinylpyrrolidone, polyvinyl alcohol, polysaccharides, and zinc oxide.

Local anesthetics may be added to reduce pain and itching sensation. Non-limiting examples of local anesthetics include benzocaine, lidocaine, dibucaine, etidocaine, benzyl alcohol, camphor, and menthol.

Non-limiting examples of emollients agents include almond oil, castor oil, ceratonia extract, cetostearoyl alcohol, cetyl alcohol, cetyl esters wax, cholesterol, cottonseed oil, cyclomethicone, ethylene glycol palmitostearate, glycerin, glycerin monostearate, glyceryl monooleate, isopropyl myristate, isopropyl palmitate, lanolin, lecithin, light mineral oil, medium-chain triglycerides, mineral oil and lanolin alcohols, petrolatum, petrolatum and lanolin alcohols, soybean oil, starch, stearyl alcohol, sunflower oil, xylitol and combinations thereof. In one embodiment, the emollients are ethylhexylstearate and ethylhexyl palmitate.

Non-limiting examples of surfactants include glyceryl monooleate, polyoxyethylene alkyl ethers, polyoxyethylene castor oil derivatives, polysorbate, sorbitan esters, benzyl alcohol, benzyl benzoate, cyclodextrins, glycerin monostearate, poloxamer, povidone and combinations thereof.

Non-limiting examples of emulsifiers include calcium stearate, carbomers, cetostearyl alcohol, cetyl alcohol, cholesterol, ethylene glycol palmitostearate, glycerin monostearate, glyceryl monooleate, lanolin, hydrous, lanolin alcohols, lecithin, and lanolin alcohols, nonionic emulsifying wax, poloxamer, poloxamers, polyoxyethylene alkyl ethers, polyoxyethylene castor oil derivatives, polyoxyethylene sorbitan fatty acid esters, polyoxyethylene stearates, propylene glycol alginate, self-emulsifying glyceryl monostearate, sodium lauryl sulfate, sorbitan esters, stearic acid, tragacanth, xanthan gum and combinations thereof.

In one embodiment, optionally in combination with one or more features of the various embodiments described above or below, the topical composition previously defined further comprises one or more cooling/refreshing agents. More particularly, the cooling/refreshing agents are selected from the group consisting of menthol ethylene glycol carbonate (Frescolat® type MGC), menthol Propylene Glycol Carbonate (Frescolat® type MPC), menthyl lactate (Frescolat ML®) and Menthone Glycerin Acetal (Frescolat MGA®); camphor, aloe vera, comfrey, cardamone, eucalyptus, peppermint, hyssop, rosemary, and combinations thereof. Even more particularly, the cooling/refreshing agents are selected from the group consisting of menthol, menthol ethylene glycol carbonate (Frescolat® type MGC), menthol Propylene Glycol Carbonate (Frescolat® type MPC), menthyl lactate (Frescolat ML®), Menthone Glycerin Acetal (Frescolat MGA®). More particularly, the cooling/refreshing agents are present in an amount from 1 to 10%, even more particularly from 1 to 5%, by weight with respect to the total weight of composition.

In another embodiment, optionally in combination with one or more features of the various embodiments described above or below, the topical composition previously defined further comprises one or more skin protectants. More particularly, the skin protectants are selected from the group consisting of symsitive (INCI name Pentylene Glycol, 4-t-Butylcyclohexanol marketed by Symrise); allantoin, cocoa butter, dimethicone, kaolin, shark liver oil, petrolatum, lanolin, vegetable oils, ethoxylated oils and lipids, polyalkylene oxides, polyvinylpyrrolidone, polyvinyl alcohol, polysaccharides, and zinc oxide.

Even more particularly, the skin protectant is symsitive (INCI name Pentylene Glycol, 4-t-Butylcyclohexanol marketed by Symrise). More particularly, the skin protectants are present in an amount from 1 to 10%, even more particularly from 1 to 5%, by weight with respect to the total weight of composition.

In one embodiment, optionally in combination with one or more features of the various embodiments described above or below, the invention relates to a topical pharmaceutical or veterinary composition in the form of a hydrogel, particularly having a viscosity from 700 to 20000 mPa·s, more particularly from 700-10000 mPa·s, and even more particularly from 700 to 1500 mPa·s as measured in a Brookfield dv-III ultra apparatus, using spindle SC4-28 at 100 rpm, atmospheric pressure, 20 °C for 10 min,

and/or has a viscosity from 200 to 10000 mPa·s, more particularly from 200-5000 mPa·s, and even more particularly from 200 to 800 mPa·s as measured in a Brookfield dv-III ultra apparatus, using spindle SC4-21 at 100 rpm, atmospheric pressure, 20 °C for 10 min,
the composition comprising:

a) a crosslinked polymer of acrylic acid, particularly carbomer 980, in an amount from 1 to 4% by weight, particularly from 1.5 to 4%, more particularly in an amount about 2% by weight, with respect to the total weight of composition;
b) a metal hydroxide selected from sodium hydroxide and potassium hydroxide, particularly potassium hydroxide, in an amount from 6 to 15% by weight, particularly from 8 to 15%, more particularly in an amount about 10% by weight, with respect to the total weight of composition;
c) a $(C_2$-$C_3)$alcohol, particularly selected from ethanol and isopropanol, particularly in an amount from 0.5 to 15 % by weight, with respect to the total weight of composition;
d) optionally one or more cooling/refreshing agents and/or skin protectants, particularly selected from the group consisting of menthol, menthol ethylene glycol carbonate (Frescolat® type MGC), menthol Propylene Glycol Carbonate (Frescolat® type MPC), menthyl lactate (Frescolat ML®), Menthone Glycerin Acetal (Frescolat MGA®), symsitive (INCI name Pentylene Glycol, 4-t-Butylcyclohexanol), and combinations thereof, particularly in an amount from 1 to 10% by weight with respect to the total weight of composition.

More particularly, in the above embodiment, the topical pharmaceutical or veterinary composition comprises a $(C_2$-$C_3)$alcohol.

In one embodiment, optionally in combination with one or more features of the various embodiments described above or below, the invention relates to a topical pharmaceutical or veterinary composition in the form of a hydrogel, particularly having a viscosity from 700 to 20000 mPa·s, more particularly from 700-10000 mPa·s, and even more particularly from 700 to 1500 mPa·s as measured in a Brookfield dv-III ultra apparatus, using spindle SC4-28 at 100 rpm, atmospheric pressure, 20 °C for 10 min,

and/or has a viscosity from 200 to 10000 mPa·s, more particularly from 200-5000 mPa·s, and even more particularly from 200 to 800 mPa·s as measured in a Brookfield dv-III ultra apparatus, using spindle SC4-21 at 100 rpm, atmospheric pressure, 20 °C for 10 min, the composition consisting of:

   a) a crosslinked polymer of acrylic acid, particularly carbomer 980, in an amount from 1 to 4% by weight, particularly from 1.5 to 4%, more particularly in an amount about 2% by weight, with respect to the total weight of composition; and
   b) a metal hydroxide selected from sodium hydroxide and potassium hydroxide, particularly potassium hydroxide, in an amount from 6 to 15% by weight, particularly from 8 to 15%, more particularly in an amount about 10% by weight, with respect to the total weight of composition;
   c) a $(C_2$-$C_3)$alcohol, particularly selected from ethanol and isopropanol, particularly in an amount from 0.5 to 15 % by weight, with respect to the total weight of composition.

In another embodiment, optionally in combination with one or more features of the various embodiments described above or below, the invention relates to a topical pharmaceutical or veterinary composition in the form of a hydrogel, particularly having a viscosity from 700 to 20000 mPa·s, more particularly from 700-10000 mPa·s, and even more particularly from 700 to 1500 mPa·s as measured in a Brookfield dv-III ultra apparatus, using spindle SC4-28 at 100 rpm, atmospheric pressure, 20 °C for 10 min,

and/or has a viscosity from 200 to 10000 mPa·s, more particularly from 200-5000 mPa·s, and even more particularly from 200 to 800 mPa·s as measured in a Brookfield dv-III ultra apparatus, using spindle SC4-21 at 100 rpm, atmospheric pressure, 20 °C for 10 min,
the composition consisting of:

   a) a crosslinked polymer of acrylic acid, particularly carbomer 980, in an amount from 1 to 4% by weight, particularly from 1.5 to 4%, more particularly in an amount about 2% by weight, with respect to the total weight of composition;
   b) a metal hydroxide selected from sodium hydroxide and potassium hydroxide, particularly potassium hydroxide, in an amount from 6 to 15% by weight, particularly from 8 to 15%, more particularly in an amount about 10% by weight, with respect to the total weight of composition;
   c) a $(C_2$-$C_3)$alcohol, particularly selected from ethanol and isopropanol, particularly in an amount from 0.5 to 15 % by weight, with respect to the total weight of composition;
   d) one or more cooling/refreshing agents and/or skin protectants, particularly selected from the group consisting of menthol, menthol ethylene glycol carbonate (Frescolat® type MGC), menthol Propylene Glycol Carbonate (Frescolat® type MPC), menthyl lactate (Frescolat ML®), Menthone Glycerin Acetal (Frescolat MGA®), symsitive (INCI name Pentylene Glycol, 4-t-Butylcyclohexanol), and combinations thereof, particularly in an amount from 1 to 10% by weight with respect to the total weight of composition.

In another embodiment, in the composition of the previous embodiments comprising components a), b) c) and d), the weight ratio between the polymer and the metal hydroxide is from 1:2 to 1:10, more particularly from 1:3 to 1:7, and even more particularly is about 1:5.
In another embodiment, in the composition of the previous embodiments comprising components a), b) c) and d), the weight ratio between the alcohol and the crosslinked polymer of acrylic acid is from 10:1 to 1:1, more particularly from 5:1 to 1:1, more particularly is about 3:2.
In another embodiment, in the composition of the previous embodiments comprising components a), b) c) and d), the weight ratio between the alcohol and the metal hydroxide is from 15:10 to 8:1, more particularly from 2:1 to 5:1, more particularly is about 10:3.
In another embodiment, optionally in combination with one or more features of the various embodiments described above or below, the weight ratio between the metal hydroxide and the alcohol is from 15:10 to 8:1, more particularly from 2:1 to 5:1, more particularly is about 10:3.
It also forms part of the invention a process for the preparation of the topical composition in the form of a hydrogel as defined above, which comprises the following steps:

   i) dissolving the metal hydroxide in water; and
   ii) adding the crosslinked polymer of acrylic acid to the solution obtained in step i).

In one embodiment, optionally in combination with one or more features of the various embodiments described above or below, steps i) and ii) are performed at room temperature.

Step ii) may be performed under stirring, for example at a speed from 50 to 15000 rpm. Generally, as it is well-known by a skilled in the art, when the homogenization is carried out at a lower speed (e.g. 50-500 rpm) it takes a longer period of time and a previous hydration treatment may be advantageous. By way of example, hydration can be performed by suspending the carbopol in water for a specific period of time (e.g. 1 day). Alternatively, when the homogenization is carried out at a higher speed (e.g. 8000-15000 rpm) it takes a shorter period of time.

In another embodiment, optionally in combination with one or more features of the various embodiments described above or below, step ii) is performed under stirring, in particular at a speed from 8000 to 15000 rpm.

In one embodiment, optionally in combination with one or more features of the various embodiments described above or below, the process as defined above comprises the following steps:

> i) dissolving the metal hydroxide in an amount from 8 to 20, particularly from 11 to 17%, particularly in an amount about 14%, by weight with respect to total weigh of the final composition in water; and
> ii) adding the crosslinked polymer of acrylic acid, particularly carbomer 980, in an amount from 1 to 4%, particularly from 1.5 to 4%, more particularly in an amount about 2%, by weight with respect to total weigh of the final composition, to the solution obtained in step i).

When further excipients are present in the composition, such as e.g. cooling/refreshing agents or skin protectants as defined above, the process comprises the additional step of adding the excipients to the mixture obtained in step ii). Thus, in one embodiment, optionally in combination with one or more features of the various embodiments described above or below, when the topical composition of the invention comprises a crosslinked polymer of acrylic acid, a metal hydroxide, water, and one or more excipients, the process for its preparation comprises the following steps:

> i) dissolving the metal hydroxide in water;
> ii) adding the crosslinked polymer of acrylic acid to the solution obtained in step i);
> iii) adding one or more excipients such as e.g. cooling/refreshing agents and/or skin protectants to the solution of step ii).

When the composition further comprises an additional solvent, such as a $(C_2-C_3)$alcohol, part of all the excipients can be alternatively first dissolved in the additional solvent and later this dissolution is added to the aqueous dissolution containing the metal hydroxide. Thus, in another embodiment, optionally in combination with one or more features of the various embodiments described above or below, when the topical composition of the invention comprises a crosslinked polymer of acrylic acid, a metal hydroxide, water, an additional solvent such as $(C_2-C_3)$alcohol, and one or more excipients such as e.g. cooling/refreshing agents and/or skin protectants as defined above, the process for its preparation comprises the following steps:

> i) dissolving the metal hydroxide in water;
> ii) adding the crosslinked polymer of acrylic acid to the solution obtained in step i);
> iii) dissolving one or more excipients such as e.g. cooling/refreshing agents and/or skin protectants in the additional solvent, such as a $(C_2-C_3)$alcohol; and
> iv) mixing the solution of step iii) with the solution of step ii).

In another embodiment, optionally in combination with one or more features of the various embodiments described above or below, the process as defined above comprises the following steps:

> i) dissolving the metal hydroxide in an amount from 8 to 20, particularly from 11 to 17%, particularly in an amount about 14%, by weight with respect to total weigh of the final composition, in water;
> ii) adding the crosslinked polymer of acrylic acid, particularly carbomer 980, in an amount from 1 to 4%, particularly from 1.5 to 4%, more particularly in an amount about 2%, by weight with respect to total weigh of the final composition, to the solution obtained in step i);
> iii) dissolving
>
> - one or more cooling/refreshing agents and/or skin protectants, particularly selected from the group consisting of menthol, menthol ethylene glycol carbonate (Frescolat® type MGC), menthol Propylene Glycol Carbonate (Frescolat® type MPC), menthyl lactate (Frescolat ML®), Menthone Glycerin Acetal (Frescolat MGA®), symsitive (INCI name Pentylene Glycol, 4-t-Butylcyclohexanol), and combinations thereof, particularly in an amount from 1 to 10% by weight with respect to the total weight of composition,

in a $(C_2-C_3)$alcohol, particularly selected from ethanol and isopropanol, in an amount from 0.5 to 15 % by weight, with respect to the total weight of composition; and iv) mixing the solution of step iii) with the solution of step ii).

In the processes described above, the amount of metal hydroxide in the final composition is lower than the amount initially dissolved in step i) because part of the metal hydroxide used for the preparation of the composition reacts with the crosslinked polymer of acrylic acid.

The invention also relates to a topical pharmaceutical or veterinary composition in the form of a hydrogel comprising:

> a) a crosslinked polymer of acrylic acid in an amount from 1 to 4% by weight with respect to the total weight of composition; and
>
> b) a metal hydroxide selected from sodium hydroxide and potassium hydroxide in an amount from 6 to 15%, more particularly from 8 to 15%, by weight with respect to the total weight of composition; obtainable by a method comprising the steps of:

> > i) dissolving the metal hydroxide in water; and
> > ii) adding the crosslinked polymer of acrylic acid to the solution obtained in step i).

In one embodiment, optionally in combination with one or more features of the various embodiments described above or below, the invention relates to a topical pharmaceutical or veterinary composition comprising:

> a) a crosslinked polymer of acrylic acid in an amount from 1 to 4% by weight with respect to the total weight of composition; and
> b) a metal hydroxide selected from sodium hydroxide and potassium hydroxide in an amount from 6 to 15%, more particularly from 8 to 15%, by weight with respect to the total weight of composition;
> c) optionally a $(C_2-C_3)$alcohol;
> d) one or more cooling/refreshing agents and/or skin protectants; obtainable by a method comprising the steps of:

> > i) dissolving the metal hydroxide in water;
> > ii) adding the crosslinked polymer of acrylic acid to the solution obtained in step i); and
> > iii) when the composition comprises a $(C_2-C_3)$alcohol, dissolving the cooling/refreshing agents and/or skin protectants in the $(C_2-C_3)$alcohol; and mixing the obtained solution with the solution of step ii), or, alternatively, when the composition does not comprises a $(C_2-C_3)$alcohol, adding the cooling/refreshing agents and/or the skin protectants to the solution of step ii).

The expression topical composition "obtainable by the process" of the invention is used herein for defining the topical composition by its preparation process and refers to the composition that can be obtained through the preparation process which comprises the steps i) and ii), or i) to iii), or i) to iv) as previously defined. For the purposes of the invention, the expressions "obtainable", "obtained" and similar equivalent expressions are used interchangeably and, in any case, the expression "obtainable" encompasses the expression "obtained".

The particular dosage to be administered of the composition of the invention will be determined by the skilled person depending on the particular circumstances of each case, including patient status, age, duration of treatment, nature of any concurrent treatment, and other factors known to the skilled person.

The compositions of the invention can be administered topically to the skin thorough a suitable device, which also forms part of the invention. The device may be any topical applicator known in the art configured to safely and effectively store and deliver the compositions of the invention to the targeted lesion to be treated. Non-limiting examples of devices that can be used include, a pen-type applicator, a roller-applicator, a sponge, a swab, a foam tipped stick, a cotton ball, a brush, a woven or non-woven fabric, roller, gauze, a glove, a flocked doe-foot applicator, or a combination thereof.

Accordingly, in one embodiment, optionally in combination with one or more features of the various embodiments described above or below, the device is selected from a pen-type applicator, a roller-applicator, a sponge, a swab, a foam tipped stick, a cotton ball, a brush, a woven or non-woven fabric, roller, gauze, a glove, and a flocked doe-foot applicator; more particularly, the device is a pen-type applicator or a roller-applicator.

As mentioned above, the composition of the invention is useful in the treatment of *Molluscum contagiosum.* Thus, the invention also relates to the topical composition as previously defined, for use as a medicament. Further, the invention relates to the topical composition as previously defined, for use in the treatment of a viral-induced lesion resulting from a viral infection.

It also forms part of the invention the use of a metal hydroxide, for the manufacture of a topical pharmaceutical or veterinary composition in the form of a hydrogel comprising:

a) a crosslinked polymer of acrylic acid in an amount from 1 to 4% by weight with respect to the total weight of composition; and

b) a metal hydroxide selected from sodium hydroxide and potassium hydroxide in an amount from 6 to 15%,more particularly from 8 to 15%, by weight with respect to the total

weight of composition, for the treatment of a viral-induced lesion resulting from a viral infection.

It also forms part of the invention a method for the treatment of a viral-induced lesion resulting from a viral infection, comprising administering an effective amount of the topical composition as previously defined above, in a subject in need thereof, including a human.

Further, the invention also relates to a medicament for the treatment of a viral-induced lesion resulting from a viral infection comprising the topical composition as previously defined.

For the purposes of the invention, the term "treatment" of the disease refers to stopping or delaying of the disease progress, when the drug is used in the subject exhibiting symptoms of disease onset. The term "prevention" refers to stopping or delaying of symptoms of disease onset, when the drug is used in the subject exhibiting no symptoms of disease onset but having high risk of disease onset.

In one embodiment, optionally in combination with one or more features of the various embodiments described above or below, the viral-induced lesion resulting from a viral infection is *Molluscum contagiosum.*

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

**Examples**

Materials

KOH (pellets ≥85% KOH, Scharlab), C980 (Lubrizol), isopropanol (Scharlab), menthol (Escude), Symsitive and Frescolat (Symrise).

General process

The compositions of the examples and comparative examples below were prepared by the process comprising the following steps:

1. Potassium hydroxide pellets were dissolved in approximately 50% of the total amount of water (Solution A);
2. (when the composition contained an alcohol): Frescolat, Symsitive and/or menthol were dissolved in isopropanol (Solution B);
3. (when the composition did not contain an alcohol): Frescolat, Symsitive and/or menthol were added to the Solution A;
4. Carbopol was added to solution A slowly and progressively while stirring constantly at 9000 rpm, to achieve a good dissolution of the Carbopol, without formation of lumps;
5. (when the composition contained an alcohol): Solution B was poured into solution A and allowed to stir until complete homogenization;
6. The remaining amount of water was added to solution A.

Examples 1-7

Following the general process described above and using the amounts indicated in the table below, the following examples were obtained. The amounts given in the table correspond to the amounts of the initial reagents by weight with respect to the total weight of composition and not to the amounts present in the final composition.

Table 1

| Example | KOH 85% (%w/w) | C980NF (%w/w) | C941NF (%w/w) | Isopropan ol (%w/w) | Gel consistency | SC4-21* |
|---|---|---|---|---|---|---|
| 1 | 14 | 2 | -- | 0 | Yes | 4400 mPa·s |
| 2 | 14 | -- | 2 | 0 | Yes | 2440 mPa·s |
| 3 | 14 | 2 | -- | 3 | Yes | 2120 mPa·s |
| 4 | 14 | -- | 2 | 3 | Yes | 2040 mPa·s |
| 5 | 16 | 2 | -- | 5 | Yes | 3600 mPa·s |
| 6 | 16 | -- | 2 | 5 | Yes | 1120 mPa·s |
| 7 | 14 | 1.47 | -- | 3 | Yes | 1560 mPa·s |
| *measured in a Brookfield dv-III ultra apparatus, using spindle SC4-21 at 100 rpm, 20 °C, atmospheric pressure, for 10 min.* | | | | | | |

Example 8

Following the general process described above and using the amounts indicated in table 2 a composition was obtained wherein the weight ratio carbopol:KOH was 1:5 (taking into account the KOH in the final composition, see table 8 below).

Table 2

| Component | Amount (weight %) |
|---|---|
| Potassium hydroxide (85%) | 14% |
| KOH (%by weight)* | 10% |
| Carbopol 980 NF | 2% |
| Isopropanol | 3% |
| Symsitive® 1690 | 3% |
| Frescolat® MGA | 2% |
| Water | q.s.100% |
| Total | 100% |
| Viscosity (SC4-28)** | 855 mPa·s |
| Gel consistency | Yes |
| *measured by titration* <br> *** measured in a Brookfield dv-III ultra apparatus, using spindle SC4-28 at 100 rpm, 20 °C, atm. pressure, 10 min.* | |

Example 9

Following the general process described above and using the amounts indicated in table 3 a composition was obtained wherein the weight ratio carbopol:KOH was 1:5 (taking into account the KOH in the final composition measured by titration).

Table 3

| Component | Amount (weight %) |
|---|---|
| Potassium hydroxide (85%) | 14% |
| KOH (%by weight)* | 10% |
| Carbopol 980 NF | 2% |
| Symsitive® 1690 | 3% |
| Frescolat® MGA | 2% |
| Water | q.s.100% |
| Total | 100% |
| Viscosity (RV7)** | 1080 mPa·s |
| Viscosity (SC4-28)** | 760 mPa·s |
| Gel consistency | Yes |
| * measured by titration<br>** measured in Brookfield dv-III ultra, using spindle SC4-28 or RV7 at 100 rpm, 20 °C, atm. pressure, 10 min. | |

Example 10

Following the general process described above and using the amounts indicated in table 4 a composition was obtained wherein the weight ratio carbopol:KOH was 1:11.7 (taking into account the KOH in the final composition measured by titration).

Table 4

| Component | Amount (weight %) |
|---|---|
| Potassium hydroxide (85%) | 15% |
| KOH (%by weight)* | 11.7% |
| Carbopol 941 | 1% |
| Symsitive® 1690 | 3% |
| Frescolat® MGA | 2% |
| Water | q.s.100% |
| Total | 100% |
| Viscosity (SC4-21)** | 236.5 mPa·s |
| Gel consistency | Yes |
| * measured by titration<br>** measured in Brookfield dv-III ultra, using spindle SC4-21 at 100 rpm, 20 °C, atm. pressure, 10 min. | |

Example 11

Following the general process described above and using the amounts indicated in table 5 a composition was obtained wherein the weight ratio carbopol:KOH was 1:1.53 (taking into account the KOH in the final composition measured by titration).

Table 5

| Component | Amount (weight %) |
|---|---|
| Potassium hydroxide (85%) | 8% |

(continued)

| Component | Amount (weight %) |
|---|---|
| KOH (%by weight)* | 6.1% |
| Carbopol 941 | 4% |
| Symsitive® 1690 | 3% |
| Frescolat® MGA | 2% |
| Water | q.s.100% |
| Total | 100% |
| Viscosity (SC4-21)** | >500 mPa·s |
| Gel consistency | Yes |
| * measured by titration<br>** measured in Brookfield dv-III ultra, using spindle SC4-21 at 100 rpm, 20 °C, atm. pressure, 10 min. | |

Comparative example 1 (Composition according to EP2471541)

Following the general process described above and using the amounts indicated in table 6 a comparative composition was obtained wherein the weight ratio carbopol:KOH was 12:1 (taking into account the KOH in the final composition measured by titration).

Table 6

| Component | Amount (weight %) |
|---|---|
| Potassium hydroxide (85%) | 4% |
| KOH (%by weight)* | 3% |
| Carbopol 941 | 0.25% |
| Menthol | 1% |
| Water | q.s.100% |
| Total | 100% |
| Viscosity (SC4-28)** | 35 mPa·s |
| Viscosity (SC4-21)** | 13.5 mPa·s |
| Gel consistency | No |
| * measured by titration<br>** measured in Brookfield dv-III ultra, using spindle SC4-28 or SC4-21 at 100 rpm, 20 °C, atm. pressure, 10 min. | |

Comparative example 2 (Composition according to CN101433617)

Following the general process described above and using the amounts indicated in table 7 a comparative composition was obtained wherein the weight ratio carbopol:KOH was 4.2:1 (taking into account the KOH in the final composition measured by titration).

Table 7

| Component | Amount (weight %) |
|---|---|
| Potassium hydroxide (85%) | 3% |
| KOH (%by weight)* | 2.1% |
| Carbopol 941 | 0.5% |
| menthol | 1% |

(continued)

| Component | Amount (weight %) |
|---|---|
| Water | q.s.100% |
| Total | 100% |
| Viscosity (SC4-28)** | 250 mPa·s |
| Viscosity (SC4-21)** | 110.5 mPa·s |
| Gel consistency | No |
| *measured by titration* <br> *\*\* measured in Brookfield dv-III ultra, using spindle SC4-28 or SC4-21 at 100 rpm, 20 °C, atm. pressure, 10 min.* | |

Gelification test

The viscosity of the formulations above was measured as indicated above. Taking into account the viscosity values, in the tables above it has been indicated if the tested formulations had the consistency of a gel or not. It was observed that formulas of the comparative examples 1 and 2 did not form a gel but showed a liquid consistency whereas the composition of examples 8 and 9 did form a gel.

Stability tests

Stability tests were performed with the formulas of examples of the invention 8 and 9 and comparative examples 1 and 2. In particular, it was evaluated whether in the tested samples KOH crystallized over time. It was observed that in the formulations of comparative examples 1 and 2, crystals of KOH could be observed from day 5, being the crystalization very evident from day 12. Furthermore, at 1 month menthol crystals were also observed. No crystallizations were observed for the examples of the invention.

Furthermore, the viscosity of the formulation of example 8 as well as the amount of KOH in % by weight was measured at time = 0 and time = 1 month.

Table 8

| Parameter | Time = 0 | Time = 1 month |
|---|---|---|
| pH | 13.011 | 13.074 |
| KOH (%by weight)* | 10% | 8.5% |
| Viscosity (SC4-21) | -- | 408.5 mPa·s |
| Viscosity (SC4-28) | 855 mPa·s | 800 mPa·s |
| Viscosity (RV7) | 1280 mPa·s | |
| *measured by titration* | | |

It was observed that the concentration of KOH did not significantly change after one month. In further stability studies it was observed that the concentration of KOH did not significally change after 15 months under storage at 25 °C, 60% RH and after 6 months under accelerated conditions (40 °C, 75% RH).

The viscosity of the formulation of examples 9-10 as well as the amount of KOH in % by weight was measured at time = 0 and time = 1 month.

Table 9

| | Time = 0 | | | Time = 1 month | | |
|---|---|---|---|---|---|---|
| | pH | KOH (%by weight)* | Viscosity (SC4-21) | pH | KOH (%by weight)* | Viscosity (SC4-21) |
| Example 9 | 12.87 | 6.1 | >500 | 12.92 | 5.9 | --- |
| Example 10 | 13.29 | 11.7 | 236.5 | 13.07 | 11.1 | 258 |
| *measured by titration* | | | | | | |

Rheologic assays: hardness, adhesiveness, cohesiveness, and elasticity

A Texturometric analysis of the hydrogel of the invention and the compositions of comparative examples 1 and 2 was performed. The following parameters: hardness, adhesiveness, cohesiveness, and elasticity were measured.

A TA-XT Plus analyzer (TA Instruments, Newcastle, UK) was used to perform the test known as TPA (Texture Profile Analysis). With this purpose, a cylindrical probe of 10 mm radius was placed at a height of 30 mm in relation to the base. The test consisted of two compression-extension cycles starting by lowering the probe at a rate of 1 mm/s, until it penetrates the sample and reaches a depth of 5 mm from the surface. Immediately, the probe rose to the initial height where it remained for 30 seconds to allow relaxation of the sample before beginning a new compression-extension cycle. During the execution of both cycles, the force/displacement curves were recorded and analyzed by the software "Exponent texture analysis" to determine the mechanical characteristics of the compositions. The hardness represents the maximum force required for the penetration of the probe into the sample and is calculated by measuring height of the first peak in the curves obtained during the first cycle. The adhesiveness is determined as the work required to extract the probe from the sample (represents the work required to overcome the attractive forces between the composition and the probe) and is calculated by determining the area under the force-displacement curve during the extension, from the beginning until the probe is separated from the composition. The cohesiveness is determined as the ratio between the positive area obtained during the understanding stage in the first and second cycles and represents the speed with which a material is reorganized after being subjected to a mechanical action (1-2). Finally, the elasticity of the composition is determined by the elastic modulus or Young's modulus which is calculated from the slope of the straight-line displacement force during the penetration stage of the probe in the sample. The calculation was performed in the two compression cycles. Approximately 20 grams of each sample were used for the analysis, and measurements were taken at a temperature of 20 °C.

The results are shown in table 10:

Table 10

| | Hardness (N) | | Adhesiveness (J) | | Cohesiveness | | Elastic modulus (1) (N/m) | | Elastic modulus (2) (N/m) | |
|---|---|---|---|---|---|---|---|---|---|---|
| | mean | SD | Mean | SD | mean | SD | mean | SD | mean | SD |
| Comp. Ex. 1 | 0.049 | 0.001 | 15.06 | 0,54 | 0.986 | 0.032 | 4.95 | 0.33 | 5.19 | 0.18 |
| Comp. Ex. 2 | 0.053 | 0.000 | 23.27 | 1.26 | 0.994 | 0.016 | 5.52 | 0.13 | 5.63 | 0.20 |
| Ex. 8 | 0.070 | 0.004 | 55.98 | 6.64 | 0.976 | 0.032 | 9.37 | 0.67 | 9.67 | 0.53 |

In order to establish differences between formulations, one-way Analysis of Variance (ANOVA) was carried out for each of the parameters except for the elasticity, for which the two-way analysis of variance was performed. As a multiple comparison test SNK (*Student—Newman—Keuls*) test was used. The ANOVA showed significant differences ($\alpha$ <0.01) between the three formulations in terms of hardness and adhesiveness. The cohesiveness was similar in all samples. The two-way ANOVA indicated no differences between the elastic modulus 1 and 2, but did show differences between the tested formulations ($\alpha$ <0.01).

In conclusion, the TPA studies indicate that RJ1 possesses greater hardness, adhesiveness and elasticity than D2 and this one than D3.

Rheologic assays: viscosity and viscoelasticity

The parameters of viscosity and viscoelasticity were determined for the hydrogel of the invention of example 8 and for the compositions of comparative examples 1 and 2.

All measurements were done in duplicate. The viscosity was measured with a cylinder geometry (C14) with a gap of 0.15 mm. The shear rate was increased and immediately decreased to potentially measure the thixotropy of the samples.

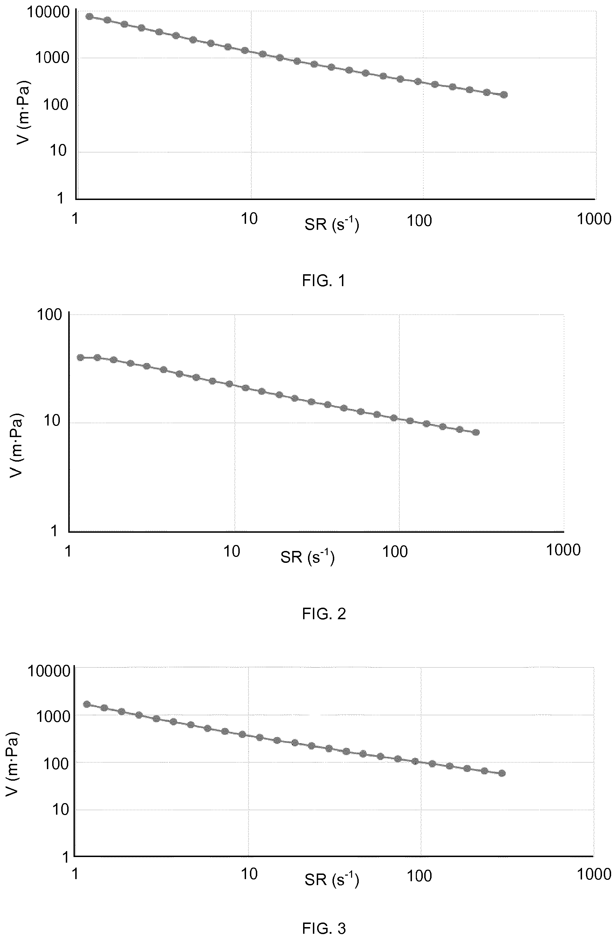

In Figures 1-3, the shear rate vs viscosity was plotted for the hydrogel of example 8 and the formulations of comparative examples 1 and 2. It was found that the viscosity of thet formulation of example 8 had about 100 times higher viscosity values than the comparative example 1 and 10 times higher than the comparative example 2 formulation.

In the table 11 below, the storage and loss modules of the formulation of example 8 and the formulations of comparative examples 1 and 2 are shown:

Table 11

| | G' | G" | δ |
|---|---|---|---|
| Example 8 | 43.3 +/- 1.63 Pa | 3.18 +/- 0.73 Pa | 4.19 +/- 0.81° |
| Comparative example 1 | 1 +- 0.89 | 1.24 +- 1.3 | 46.46 +- 24.94° |
| Comparative example 2 | 7.29 +/- 0.78 Pa | 1.46 +/- 0.04 Pa | 11.37 +/- 1.48° |

In the formulation of example 8 the values are quite small overall. The storage module (G') value, dominated the loss module (G") value, which indicates an elastic behaviour. At 0.010 Pa strain, the storage module had the value 43.3 +/- 1.63 Pa, the loss module 3.18 +/- 0.73 Pa. The viscoelastic properties of the comparative example 1 were measured and the data was found to be too unreliable. Due to the low viscosity of the sample, the rheometer could not give an accurate reading of the values and a linear viscoelastic region was not detected. The comparative example 2 had smaller module values as compared to the formulation of example 8 that made the measurement less accurate than for the formulation of example 8. The measurement was towards the lower limit of the instrument range. The storage module was larger than the loss module. However, both values were very small. At 0.0117 Pa strain, the storage module (G') had the value 7.29 +/- 0.78 Pa, the loss module (G") 1.46 +/- 0.04 Pa.

The phase angles for the three different formulations were also measured. The phase angle of the formulation of example 8 was 4.19 +/- 0.81°. The phase angle for the formulation of comparative example 2 was 11.37 +/- 1.48°. The value could not be measured for the formulation of comparative example 1 given the low viscosity of the sample.

Clinical study

An observational, prospective, multicenter, not controlled study was carried out with the aim to evaluate the efficacy of the topical application of Example 8 for the treatment of Molluscum Contagiosum (MC) in pediatric people, aged two and over, through the SUS (System Usability Scale).

The open, not controlled, one-arm study consisted in two visits (basal and 15 days after) in which 32 patients participated. The composition was applied once a day (always at night) and the application was repeated daily in the affected areas, unlike previous studies which demonstrated the benefit of a twice-daily application.

The obtained results showed an efficacy on complete cure in MC lesions of 75% of patients, 9.4% of them showed a significant improvement (disappearance of 75% of injuries). There was also a 84.4% of reduction in the number of injuries, from an initial average number of 11.2 per patient to a final mean number of injuries of 2.0. The time of cure was also significant: 5 days to cure in comparison with previous literature in which the average time to clearance is much higher.

Despite all patients presented Adverse Events (AEs), just 15.6% showed Serious Adverse Events (SAEs). Between the usual AEs with a moderate severity were scratching (38.71%) and scab (40%).

## Claims

1. A topical pharmaceutical or veterinary composition in the form of a hydrogel comprising:

   a) a crosslinked polymer of acrylic acid in an amount from 1 to 4% by weight with respect to the total weight of composition; and
   b) a metal hydroxide selected from sodium hydroxide and potassium hydroxide in an amount from 6 to 15% by weight with respect to the total weight of composition.

2. The topical pharmaceutical or veterinary composition according to claim 1, wherein the metal hydroxide is present in an amount from 8 to 15% by weight with respect to the total weight of composition.

3. The topical pharmaceutical or veterinary composition according to any of the claims 1-2, wherein the viscosity of the composition is from more particularly from 700-10000 mPa·s as measured in a Brookfield dv-III ultra apparatus, using spindle SC4-28 at 100 rpm, atmospheric pressure, 20 °C for 10 min, and/or is from 200 to 200-5000 mPa·s as measured in a Brookfield dv-III ultra apparatus, using spindle SC4-21 at 100 rpm, atmospheric pressure, 20 °C for 10 min,.

4. The topical pharmaceutical or veterinary composition according to any of the claims 1-3, wherein the polymer of acrylic acid is crosslinked with allyl sucrose or allyl pentaerythritol.

5. The topical pharmaceutical or veterinary composition according to claim 4, wherein the crosslinked polymer of acrylic acid is carbomer 980.

6. The topical pharmaceutical or veterinary composition according to any of the claims 1-5, wherein the crosslinked polymer of acrylic acid is present in an amount about 2% by weight with respect to the total weight of composition.

7. The topical pharmaceutical or veterinary composition according to any of the claims 1-6, wherein the metal hydroxide is potassium hydroxide.

8. The topical pharmaceutical or veterinary composition according to any of the claims 1-7, wherein the metal hydroxide is present in an amount from 9 to 12% by weight with respect to the total weight of composition.

9. The topical pharmaceutical or veterinary composition according to any of the claims 1-8, wherein the weight ratio between the metal hydroxide and the crosslinked polymer of acrylic acid is more particularly from 1:3 to 1:7.

10. The topical pharmaceutical or veterinary composition according to any of the claims 1-9, which further comprises a $(C_2\text{-}C_3)$alcohol.

11. The topical pharmaceutical or veterinary composition according to claim 10, wherein the weight ratio between the alcohol and the crosslinked polymer of acrylic acid is from 5:1 to 1:1.

12. The topical pharmaceutical or veterinary composition according to any of the claims 1-11, which further comprises additional pharmaceutically or veterinary acceptable excipients.

13. The topical pharmaceutical or veterinary composition according to claim 12, wherein the excipients comprise one or more cooling/refreshing agents.

14. A device comprising the topical pharmaceutical or veterinary composition according to any of the claims 1-13.

15. A topical pharmaceutical or veterinary composition as defined in any of the claims 1-13, for use in the treatment of a viral-induced lesion resulting from a viral infection.

16. The topical pharmaceutical or veterinary composition for use according to claim 15, wherein the viral-induced lesion resulting from a viral infection is *Molluscum contagiosum.*

**Patentansprüche**

1. Eine topische pharmazeutische oder tiermedizinische Zusammensetzung in Form eines Hydrogels, umfassend:

   a) ein vernetztes Polymer von Acrylsäure in einer Menge von 1 bis 4 Gew.-%, bezogen auf das gesamte Gewicht der Zusammensetzung; und
   b) ein Metallhydroxid, ausgewählt aus Natriumhydroxid und Kaliumhydroxid, in einer Menge von 6 bis 15 Gew.-%, bezogen auf das gesamte Gewicht der Zusammensetzung.

2. Die topische pharmazeutische oder tiermedizinische Zusammensetzung nach Anspruch 1, wobei das Metallhydroxid in einer Menge von 8 bis 15 Gew.-%, bezogen auf das gesamte Gewicht der Zusammensetzung, vorhanden ist.

3. Die topische pharmazeutische oder tiermedizinische Zusammensetzung nach einem der Ansprüche 1 bis 2, wobei die Viskosität der Zusammensetzung insbesondere von 700 bis 10000 mPa·s, gemessen in einem Brookfield dv-III ultra-Gerät, unter Verwendung der Spindel SC4-28 bei 100 U/min, Atmosphärendruck, 20 °C für 10 min, und/oder von 200 bis 200 bis 5000 mPa·s, gemessen in einem Brookfield dv-III ultra-Gerät, unter Verwendung der Spindel SC4-21 bei 100 U/min, Atmosphärendruck, 20 °C für 10 min, beträgt.

4. Die topische pharmazeutische oder tiermedizinische Zusammensetzung nach einem der Ansprüche 1 bis 3, wobei das Polymer von Acrylsäure mit Allylsaccharose oder Allylpentaerythrit vernetzt ist.

**5.** Die topische pharmazeutische oder tiermedizinische Zusammensetzung nach Anspruch 4, wobei das vernetzte Polymer von Acrylsäure Carbomer 980 ist.

**6.** Die topische pharmazeutische oder tiermedizinische Zusammensetzung nach einem der Ansprüche 1 bis 5, wobei das vernetzte Polymer von Acrylsäure in einer Menge von etwa 2 Gew.-%, bezogen auf das gesamte Gewicht der Zusammensetzung, vorhanden ist.

**7.** Die topische pharmazeutische oder tiermedizinische Zusammensetzung nach einem der Ansprüche 1 bis 6, wobei das Metallhydroxid Kaliumhydroxid ist.

**8.** Die topische pharmazeutische oder tiermedizinische Zusammensetzung nach einem der Ansprüche 1 bis 7, wobei das Metallhydroxid in einer Menge von 9 bis 12 Gew.-%, bezogen auf das gesamte Gewicht der Zusammensetzung, vorhanden ist.

**9.** Die topische pharmazeutische oder tiermedizinische Zusammensetzung nach einem der Ansprüche 1 bis 8, wobei das Gewichtsverhältnis von dem Metallhydroxid zu dem vernetzten Polymer von Acrylsäure insbesondere 1:3 bis 1:7 beträgt.

**10.** Die topische pharmazeutische oder tiermedizinische Zusammensetzung nach einem der Ansprüche 1 bis 9, die weiterhin einen $(C_2$-$C_3)$-Alkohol umfasst.

**11.** Die topische pharmazeutische oder tiermedizinische Zusammensetzung nach Anspruch 10, wobei das Gewichtsverhältnis von dem Alkohol zu dem vernetzten Polymer von Acrylsäure von 5:1 bis 1:1 beträgt.

**12.** Die topische pharmazeutische oder tiermedizinische Zusammensetzung nach einem der Ansprüche 1 bis 11, die weiterhin zusätzliche pharmazeutisch oder tiermedizinisch akzeptable Hilfsstoffe umfasst.

**13.** Die topische pharmazeutische oder tiermedizinische Zusammensetzung nach Anspruch 12, wobei die Hilfsstoffe ein oder mehrere Kühl-/Erfrischungsmittel umfassen.

**14.** Eine Vorrichtung umfassend die topische pharmazeutische oder tiermedizinische Zusammensetzung nach einem der Ansprüche 1 bis 13.

**15.** Eine topische pharmazeutische oder tiermedizinische Zusammensetzung wie in einem der Ansprüche 1 bis 13 definiert zur Verwendung bei der Behandlung von einer virusinduzierten Läsion, die aus einer viralen Infektion resultiert.

**16.** Die topische pharmazeutische oder tiermedizinische Zusammensetzung zur Verwendung nach Anspruch 15, wobei die virusinduzierte Läsion, die aus einer viralen Infektion resultiert, *Molluscum contagiosum* ist.

## Revendications

**1.** Une composition pharmaceutique ou vétérinaire topique sous forme d'hydrogel comprenant:

a) un polymère réticulé d'acide acrylique en une quantité allant de 1 à 4 % en poids par rapport au poids total de la composition; et
b) un hydroxyde de métal choisi parmi l'hydroxyde de sodium et l'hydroxyde de potassium en une quantité allant de 6 à 15 % en poids par rapport au poids total de la composition.

**2.** La composition pharmaceutique ou vétérinaire topique selon la revendication 1, dans laquelle l'hydroxyde de métal est présent en une quantité allant de 8 à 15 % en poids par rapport au poids total de la composition.

**3.** La composition pharmaceutique ou vétérinaire topique selon l'une quelconque des revendications 1 à 2, dans laquelle la viscosité de la composition est plus en particulier de 700 à 10000 mPa·s, telle que mesurée dans un appareil Brookfield dv-III ultra, en utilisant la broche SC4-28 à 100 tr/min, sous pression atmosphérique, à 20 °C pendant 10 min, et/ou est de 200 à 5000 mPa·s, telle que mesurée dans un appareil Brookfield dv-III ultra, en utilisant la broche SC4-21 à 100 tr/min, sous pression atmosphérique, à 20 °C pendant 10 min.

4. La composition pharmaceutique ou vétérinaire topique selon l'une quelconque des revendications 1 à 3, dans laquelle le polymère d'acide acrylique est réticulé avec de l'allyl saccharose ou de l'allyl pentaérythritol.

5. La composition pharmaceutique ou vétérinaire topique selon la revendication 4, dans laquelle le polymère réticulé d'acide acrylique est le carbomère 980.

6. La composition pharmaceutique ou vétérinaire topique selon l'une quelconque des revendications 1 à 5, dans laquelle le polymère réticulé d'acide acrylique est présent en une quantité d'environ 2 % en poids par rapport au poids total de la composition.

7. La composition pharmaceutique ou vétérinaire topique selon l'une quelconque des revendications 1 à 6, dans laquelle l'hydroxyde de métal est l'hydroxyde de potassium.

8. La composition pharmaceutique ou vétérinaire topique selon l'une quelconque des revendications 1 à 7, dans laquelle l'hydroxyde de métal est présent en une quantité de 9 à 12 % en poids par rapport au poids total de la composition.

9. La composition pharmaceutique ou vétérinaire topique selon l'une quelconque des revendications 1 à 8, dans laquelle le rapport de poids de l'hydroxyde de métal au polymère réticulé d'acide acrylique est plus en particulier de 1:3 à 1:7.

10. La composition pharmaceutique ou vétérinaire topique selon l'une quelconque des revendications 1 à 9, qui comprend en outre un alcool en (C$_2$-C$_3$).

11. La composition pharmaceutique ou vétérinaire topique selon la revendication 10, dans laquelle le rapport de poids de l'alcool au polymère réticulé d'acide acrylique est de 5:1 à 1:1.

12. La composition pharmaceutique ou vétérinaire topique selon l'une quelconque des revendications 1 à 11, qui comprend en outre des excipients pharmaceutiquement ou vétérinaires acceptables additionnels.

13. La composition pharmaceutique ou vétérinaire topique selon la revendication 12, dans laquelle les excipients comprennent un ou plusieurs agents de refroidissement/rafraîchissement.

14. Un dispositif comprenant la composition pharmaceutique ou vétérinaire topique selon l'une quelconque des revendications 1 à 13.

15. Une composition pharmaceutique ou vétérinaire topique telle que définie dans l'une quelconque des revendications 1 à 13, destinée à être utilisée dans le traitement d'une lésion induite par un virus résultant d'une infection virale.

16. La composition pharmaceutique ou vétérinaire topique pour l'utilisation selon la revendication 15, dans laquelle la lésion induite par un virus résultant d'une infection virale est *Molluscum contagiosum.*

FIG. 1

FIG. 2

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2471541 A
- CN 101433617